(19) Europäisches Patentamt
European Patent Office
Office européen des brevets

(11) Veröffentlichungsnummer: **0 006 501**
**B1**

# (12) EUROPÄISCHE PATENTSCHRIFT

(45) Veröffentlichungstag der Patentschrift: **08.06.83**

(51) Int. Cl.³: **C 07 C 1/20**, C 07 C 11/02

(21) Anmeldenummer: **79101817.9**

(22) Anmeldetag: **08.06.79**

(54) **Herstellung von Olefinen aus Methanol bzw. Dimethyläther.**

(30) Priorität: **22.06.78 DE 2827385**

(43) Veröffentlichungstag der Anmeldung: **09.01.80 Patentblatt 80/1**

(45) Bekanntmachung des Hinweises auf die Patenterteilung: **08.06.83 Patentblatt 83/23**

(84) Benannte Vertragsstaaten: **BE DE FR GB NL**

(56) Entgegenhaltungen:
**DE-A-2052782**
**US-A-4083888**
**US-A-4083889**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Marosi, Laszlo, Dr., Londoner Ring 11, D-6700 Ludwigshafen (DE)**
Erfinder: **Stabenow, Joachim, Dr., Am Feldrain 22, D-6940 Weinheim (DE)**
Erfinder: **Schwarzmann, Matthias, Dr., Carl-Bosch-Strasse 54, D-6703 Limburgerhof (DE)**

**EP 0 006 501 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Herstellung von Olefinen aus Methanol bzw. Dimethyläther

In der DE-OS 2438252 ist die Umwandlung von Methanol bzw. Dimethyläther in aromatische Verbindungen mit Hilfe von Zeolithkatalysatoren beschrieben, wobei als Ausgangsmaterial auch Rohmethanol verwendet werden kann. Durch Erhöhung der Belastung des Katalysators kommt man zu überwiegend gasförmigen Reaktionsprodukten, die auch Olefine enthalten. Wegen der ungünstigen Produktzusammensetzung des erhaltenen Gasgemisches und wegen der verfahrenstechnischen Nachteile ist jedoch eine wirtschaftliche Herstellung von Olefinen, wie Äthylen, Propylen und Butylen, auf diese Weise nicht möglich. Insbesondere beträgt der Anteil des Äthylens im Umsetzungsprodukt nur etwa 8 bis 10%. Mit der Erhöhung der Belastung ist ausserdem ein starker Rückgang des Umsatzes verbunden, und die Abtrennung der gebildeten Kohlenwasserstoffe vom nicht umgesetzten Dimethyläther ist technisch aufwendig. Auch die grosse Wärmetönung der Reaktion verursacht verfahrenstechnische Probleme.

Aus der DE-OS 2615150 ist bekannt, Methanol bzw. Dimethyläther bei unteratmosphärischem Partialdruck oder durch Verdünnung des Zufuhrmaterials mit Wasserdampf, Stickstoff, Wasserstoff, Kohlendioxid oder einem Paraffin mit bis zu 5 Kohlenstoffatomen umzusetzen. Dabei wird als Katalysator der Zeolith H-ZSM-5 eingesetzt, der vorzugsweise ein $SiO_2/Al_2O_3$-Verhältnis von wenigstens 70 aufweisen soll. Als Hauptprodukt entsteht bei der Umsetzung Propylen.

Es war die Aufgabe gestellt, ein Verfahren zu entwickeln, bei dem die Umsetzung von Methanol bzw. Dimethyläther zu besseren Ausbeuten an $C_2-C_4$-Olefinen führt und insbesondere der Äthylenanteil im Reaktionsprodukt erhöht wird.

Es wurde nun gefunden, dass man bei der Herstellung von Olefinen durch Umsetzung von Methanol und/oder Dimethyläther in Gegenwart von zeolithhaltigen Katalysatoren des Typs H-ZSM-5, die ein $SiO_2/Al_2O_3$-Molverhältnis von kleiner als 70 aufweisen in der Gasphase bei Temperaturen von 250 bis 600°C, vorzugsweise zwischen 330 bis 480°C und bei einem Druck von Normaldruck bis etwa 30 bar, verbesserte Ausbeuten an Olefinen, und insbesondere eine Erhöhung des Äthylenanteils erzielt, wenn man den Ausgangsstoffen vor der Umsetzung mindestens 50 Gew.-% Wasser zusetzt und die Belastung des Katalysators so wählt, dass die Stoffe vollständig umgesetzt werden.

Aus der US-A-4083889 ist auch schon ein Verfahren zur Umwandlung von Methanol in Gegenwart von zeolithhaltigen Katalysatoren des Typs H-ZSM-5 bekannt, bei dem Wasser als Verdünnungsmittel verwendet und im Bereich von beispielsweise 50 bis 65% Umsatz an Methanol gearbeitet werden muss, um entsprechend günstige Äthylenausbeuten zu erhalten.

Eine zweckmässige Ausführungsform besteht darin, dass man als Verdünnungsmittel 50 bis 80 Gew.-% Wasser verwendet. In dieser Wassermenge ist das durch Dehydratisierung von Methanol bzw. Dimethyläther nach der folgenden Gleichung entstehende Wasser nicht enthalten:

$$2CH_3OH \leftrightarrows CH_3{-}O{-}CH_3 + H_2O \xrightarrow{Kat.} (CH_2)_x + 2\,H_2O$$

Der Wassergehalt wird auf Methanol bzw. bei Verwendung von Dimethyläther auf die nach Gleichung äquivalente Menge Methanol bezogen.

Dieses Ergebnis war überraschend, da Zeolithe im allgemeinen gegen Wasserdampf nicht beständig sind, so dass durch hohe Wasserdampfkonzentrationen eine Zerstörung der Kristallstruktur und damit eine rasche Inaktivierung des Katalysators zu erwarten war. Es ist weiterhin überraschend, dass der Anteil an Äthylen im Reaktionsprodukt wesentlich höher liegt als bei einer entsprechenden Erhöhung der Belastung.

Erfindungsgemäss wird die oben beschriebene Beschickung bei Temperaturen zwischen 250 bis 600°C, vorzugsweise zwischen 330 bis 480°C, und bei einem Druck von Normaldruck bis etwa 30 bar mit einem zeolithhaltigen Katalysator in Berührung gebracht. Es werden dabei vorzugsweise Zeolithe der ZSM-Gruppe, insbesondere der Zeolith H-ZSM-5 als Katalysator eingesetzt. Es ist zur Erzielung einer hinreichend hohen Aktivität erforderlich, dass der Zeolith teilweise oder ganz in seiner Wasserstofform vorliegt. Besonders bevorzugt wird daher der Zeolithe H-ZSM-5 als Katalysator.

Bei der Durchführung des erfindungsgemässen Verfahrens ist es vorteilhaft, wenn der Zeolith H-ZSM-5 ein $SiO_2/Al_2O_3$-Molverhältnis von kleiner 70, insbesondere kleiner als 40 aufweist.

Die Belastung des Katalysators, ausgedrückt in g Methanol oder Dimethyläther/g Kat./h, wird vorteilhafterweise so gewählt, dass diese Stoffe möglichst vollständig umgesetzt werden, damit keine Abtrenn- und Rückführprobleme entstehen. Es können jedoch ohne Nachteil für die Zusammensetzung des entstehenden Kohlenwasserstoffgemisches auch höhere Belastungen gewählt werden. Ein weiteres Kriterium für die optimale Belastung ist durch die Verkohlung des Katalysators gegeben. Je nach Betriebsbedingungen muss die

Arbeitszeit des Katalysators auf die Zeit, die für die Regenerierung mit Luft, Sauerstoff oder Luft und Wasser-Mischungen erforderlich ist, abgestellt werden. Im allgemeinen wird deshalb die Belastung etwa in dem Bereich von 0,5 bis 100 g Methanol (Dimethyläther)/g Zeolith/h liegen.

Es ist ein weiterer Vorteil des erfindungsgemässen Verfahrens, dass auch ein 100%iger Umsatz von Methanol bzw. Dimethyläther erreicht werden kann, ohne dass nennenswerte Mengen höhere Kohlenwasserstoffe (Öl) entstehen. Dies war mit Hilfe der bisher bekannten Verfahren nicht möglich. Darin zeigt sich auch die spezifische Wirkung von Wasser gemäss der Erfindung. Dieser Effekt ist weder durch eine noch so hohe Belastung des Katalysators, noch durch die Verwendung anderer Verdünnungsmittel auf eine ähnlich vorteilhafte Weise zu erzielen. Versuche, anstelle von Wasser beispielsweise $N_2$ als Verdünnungsmittel für Methanoldampf oder Dimethyläther zu verwenden und so bei 100% Umsatz überwiegend Äthylen zu erhalten, führen unter vergleichbaren Bedingungen zu schlechteren Ergebnissen.

Die Durchführung des erfindungsgemässen Verfahrens und der Einfluss der prozessvariablen wird anhand der nachstehenden Beispiele näher erläutert.

Als Reaktor war ein Durchflussreaktor mit 20 mm Durchmesser benutzt, die Reaktionsprodukte wurden gaschromatographisch analysiert.

In den Versuchen werden 20 g Katalysator in Form von Strängen mit 1 mm Durchmesser eingesetzt. Der Gehalt der Stränge an H-ZSM-5 Zeolith beträgt 65 Gew.-%, das Molverhältnis $SiO_2/Al_2O_3$ im Zeolith beträgt 33.

Beispiel 1

Dieses Beispiel zeigt den Einfluss von Wasser auf die Produktzusammensetzung bei der Umsetzung von Methanol. Die Reaktionsbedingungen und Versuchsergebnisse sind in der folgenden Tabelle zusammengefasst. Der Katalysator wurde nach jedem Versuch durch Hinüberleiten von Luft bei 500 bis 550°C regeneriert.

| | | a) | b) |
|---|---|---|---|
| Eingangstemperatur | | 400° C | 400° C |
| Temperaturanstieg | | 120° C | 100° C |
| Druck | | 1,15 bar | 1,23 bar |
| Beschickung | $CH_3OH$ | 100 | 62 |
| | $H_2O$ | 0 | 180 |
| Belastung | | 60 g/h | 300 g/h |
| Gesamtbelastung (Methanol) | | 360 g | 160 g |
| Umsetzung | | 100% | 100% |

Zusammensetzung der Reaktionsprodukte

| | | | |
|---|---|---|---|
| Öl | | 32 g | 0,58 g |
| Gasförmige Produkte (Vol.-%) | | | |
| Olefine | $C_2$ | 18 | 40 |
| | $C_3$ | 24 | 25 |
| | $C_4$ | 27 | 9 |
| Paraffine | $C_1$ | 8 | 7 |
| | $C_2$ | – | – |
| | $C_3$ | 8 | 4 |
| | $C_4$ | 12 | 6 |

Beispiel 2

Dieses Beispiel zeigt den Einfluss von Wasser auf die Produktzusammensetzung bei der Umsetzung von Dimethyläther.

| | | a) | b) |
|---|---|---|---|
| Eingangstemperatur | | 400° C | 400° C |
| Temperaturanstieg | | 50° C | 90° C |
| Druck | | 1,2 bar | 1,2 bar |
| Beschickung | Dimethyläther | 100 | 44 g |
| | $H_2O$ | – | 220 g |
| Belastung | | 23 g/h | 260 g/h |
| Gesamtbelastung (Dimethyläther) | | 110 g | 100 g |
| Umsetzung | | 100% | 100% |

Zusammensetzung der Reaktionsprodukte

| | | | |
|---|---|---|---|
| Öl | | 28,2 g | 3,22 g |
| Gasförmige Produkte (Vol.-%) | | | |
| Olefine | $C_2$ | 9 | 38 |
| | $C_3$ | 10 | 34 |
| | $C_4$ | 11 | 13 |
| Paraffine | $C_1$ | 30 | 4 |
| | $C_2$ | – | 0,2 |
| | $C_3$ | 18 | 6 |
| | $C_4$ | 17 | 4 |

Beispiel 3

Dieses Beispiel zeigt den Einfluss der Belastung des Katalysators mit Methanol-Wasser-Gemischen auf die Produktzusammensetzung.

| | | a) | b) | c) |
|---|---|---|---|---|
| Eingangstemperatur | | 400° C | 400° C | 400° C |
| Temperaturanstieg | | 20° C | 25° C | 60° C |
| Druck | | 1,2 bar | 1,2 bar | 1,2 bar |
| Beschickung | Methanol | 32 | 32 | 32 |
| | $H_2O$ | 180 | 180 | 180 |
| Belastung | | 50 g/h | 100 g/h | 300 g/h |
| Gesamtbelastung (Methanol) | | 50 g | 60 g | 120 g |
| Umsetzung | | 100% | 100% | 100% |

Zusammensetzung der Reaktionsprodukte

| | | a) | b) | c) |
|---|---|---|---|---|
| Öl | | – | 0,1 g | 0,15 g |
| Gasförmige Produkte (Vol.-%) | | | | |
| Olefine | $C_2$ | 35 | 40 | 45 |
| | $C_3$ | 28 | 25 | 25 |
| | $C_4$ | 18 | 15 | 10 |
| Paraffine | $C_1$ | 3 | 4 | 4 |
| | $C_2$ | – | – | – |
| | $C_3$ | 5 | 5 | 4 |
| | $C_4$ | 8 | 7 | 6 |

Beispiel 4

Dieses Beispiel zeigt den Einfluss der Temperatur auf die Produktzusammensetzung bei der Umsetzung von Methanol-Wasser-Gemischen.

| | | a) | b) | c) |
|---|---|---|---|---|
| Eingangstemperatur | | 330° C | 400° C | 400° C |
| Temperaturanstieg | | 40° C | – | 50° C |
| Druck | | 1,2 bar | 1,2 bar | 1,2 bar |
| Beschickung | Methanol | 32 | 32 | 32 |
| | Wasser | 180 | 180 | 180 |
| Belastung | | 300 g/h | 300 g/h | 300 g/h |
| Gesamtbelastung (Methanol) | | 210 g | 120 g | 160 g |
| Umsetzung | | 100% | 100% | 100% |

Zusammensetzung der Reaktionsprodukte

| | | a) | b) | c) |
|---|---|---|---|---|
| Öl | | 0,48 g | 0,15 g | 1,15 g |
| Gasförmige Produkte (Vol.-%) | | | | |
| Olefine | $C_2$ | 48 | 45 | 30 |
| | $C_3$ | 21 | 25 | 30 |
| | $C_4$ | 7 | 12 | 17 |
| Paraffine | $C_1$ | 2 | 4 | 8 |
| | $C_2$ | – | – | – |
| | $C_3$ | 5 | 4 | 4 |
| | $C_4$ | 8 | 6 | 9 |

Beispiel 5

Dieses Beispiel zeigt den Einfluss der Wasserkonzentration auf die Produktzusammensetzung bei der Umsetzung von Methanol-Wasser-Gemischen.

| | | a) | b) | c) | d) | e) |
|---|---|---|---|---|---|---|
| Eingangstemperatur | | 400° C | 330° C | 330° C | 330° C | 400° C |
| Temperaturanstieg | | 35° C | 120° C | 95° C | 40° C | 100° C |
| Druck | | 1,15 bar | 1,3 bar | 1,2 bar | 1,2 bar | 1,23 bar |
| Beschickung | Methanol | 20 | 136 | 100 | 32 | 62 |
| | Wasser | 180 | 180 | 180 | 180 | 180 |
| Gesamtbelastung (Methanol) | | 150 g | 230 g | 210 g | 210 g | 160 g |
| Umsetzung | | 100% | 100% | 100% | 100% | 100% |

Zusammensetzung der Reaktionsprodukte

| | | | | | | |
|---|---|---|---|---|---|---|
| Öl | | 0,38 g | 0,1 g | 0,1 g | 0,48 g | 0,58 g |
| Gasförmige Produkte (Vol.-%) | | | | | | |
| Olefine | $C_2$ | 48 | 30 | 35 | 48 | 40 |
| | $C_3$ | 25 | 30 | 25 | 21 | 25 |
| | $C_4$ | 8 | 20 | 22 | 7 | 9 |
| Paraffine | $C_1$ | 4 | 2 | 2 | 2 | 7 |
| | $C_2$ | – | – | – | – | – |
| | $C_3$ | 5 | 3 | 4 | 5 | 4 |
| | $C_4$ | 7 | 8 | 8 | 8 | 6 |

Beispiel 6

Dieses Beispiel veranschaulicht die spezifische Wirkung von Wasserdampf im Vergleich zu anderen Gasen. Für diesen Versuch wurden die Gaschromatogramme nur nach den Höhen der einzelnen Komponenten ausgewertet, deren relatives Verhältnis in der folgenden Tabelle angegeben ist. Für diesen Versuch wurde eine Mischung von 35 g Dimethyläther und 180 g Wasser pro Stunde über den Katalysator geleitet. In den Versuchen b, c und d wurde eine bei der Reaktionstemperatur volumengleiche Gasmenge in Mischung mit Dimethyläther eingesetzt.

Relative Peakhöhen

| Mischgas | Äthylen | Propylen | Butene | Methan | Propan | Buten |
|---|---|---|---|---|---|---|
| $H_2O$ | 74 | 20 | 0,0015 | 0,0009 | 0,05 | 0,003 |
| $CO_2$ | 54 | 27 | 0,05 | 0,0007 | 0,08 | 0,05 |
| $N_2$ | 39 | 33 | 0,06 | 0,03 | 0,11 | 0,07 |
| He | 37 | 37 | 0,08 | 0,04 | 0,09 | 0,06 |

Beispiel 7

Nachdem mit dem beschriebenen Katalysator die vorstehend angeführten Versuche durchgeführt worden sind, wurde er wieder nach Beispiel 1 getestet, um seine Beständigkeit zu prüfen.

| | | |
|---|---|---|
| Eingangstemperatur | | 400° C |
| Temperaturanstieg | | 100° C |
| Druck | | 1,2 bar |
| Beschickung | Methanol | 62 |
| | Wasser | 180 |
| Belastung | | 300 g/h |
| Gesamtbelastung (Dimethyläther) | | 160 g Methanol |
| Umsetzung | | 100% |

Zusammensetzung der Reaktionsprodukte

| | | |
|---|---|---|
| Öl | | 0,70 g |
| Gasförmige Produkte (Vol.-%) | | |
| Olefine | $C_2$ | 43 |
| | $C_3$ | 22 |
| | $C_4$ | 8 |
| Paraffine | $C_1$ | 8 |
| | $C_2$ | – |
| | $C_3$ | 4 |
| | $C_4$ | 6 |

**Patentanspruch**

Verfahren zur Herstellung von Olefinen, insbesondere mit einem hohen Anteil an Ethylen durch Umsetzung von Methanol und/oder Dimethyläther in Gegenwart von zeolithhaltigen Katalysatoren des Typs H-ZSM-5 in der Gasphase, bei Temperaturen von 250 bis 600°C, vorzugsweise zwischen 330 bis 480°C und einem Druck bis etwa 30 bar unter Zusatz von mindestens 50 Gew.-% Wasser zu den Ausgangsstoffen vor der Umsetzung, dadurch gekennzeichnet, dass man einen zeolithhaltigen Katalysator verwendet, der ein $SiO_2/Al_2O_3$-Molverhältnis von 70 aufweist und die Belastung des Katalysators so wählt, dass die Stoffe vollständig umgesetzt werden.

**Claim**

A process for the preparation of olefins, especially olefins with a large proportion of ethylene, by conversion of methanol and/or dimethyl ether in the gas phase, at a temperature of from 250 to 600°C, preferably at from 330 to 480°C, and at a pressure of up to about 30 bars, over a zeolite-containing catalyst of the H-ZSM-5 type, at least 50% by weight of water being added to the starting materials before the conversion, wherein a zeolite-containing catalyst is used which has an $SiO_2/Al_2O_3$ molar ratio of less than 70, and the throughput is selected so as to give complete conversion of the starting materials.

**Revendication**

Procédé de préparation d'oléfines, en particulier de l'éthylène, par transformation du méthanol et(ou) de l'éther diméthylique en phase gazeuse, à des températures comprises entre 250 et 600°C et de préférence entre 330 et 480°C et sous une pression pouvant aller jusqu'à environ 30 bars, en présence d'un catalyseur zéolithique du type H-ZSM-5 et avec addition, avant la réaction, d'au moins 50% en poids d'eau aux substances de départ, caractérisé en ce que la réaction est réalisée en présence d'un catalyseur zéolithique avec un rapport molaire $SiO_2/Al_2O_3$ inférieur à 70 et en choisissant la charge du catalyseur de telle façon que la transformation des produits de départ soit complète.